# EUROPEAN PATENT SPECIFICATION

(11) **EP 2 473 493 B1**
(45) Date of publication and mention of the grant of the patent: **30.04.2014**
(21) Application number: 10814414.8
(22) Date of filing: 01.09.2010
(51) Int. Cl.: A61K 31/445, C07D 307/48, A61P 31/20

(54) **METHODS OF TREATING POXVIRAL INFECTIONS**
VERFAHREN ZUR BEHANDLUNG VON POXVIRUSINFEKTIONEN
PROCÉDÉS DE TRAITEMENT D'INFECTIONS POXVIRALES

(30) Priority: 04.09.2009 US 272252 P
(43) Date of publication of application: 11.07.2012
(73) Proprietor: United Therapeutics Corporation, Silver Spring, MD 20910 (US); THE CHANCELLOR, MASTERS AND SCHOLARS OF THE UNIVERSITY OF OXFORD, Oxford OX1 2JD (GB)
(72) Inventor: RAMSTEDT, Urban, Silver Spring, MD 20910 (US); KLOSE, Brennan, Silver Spring, MD 20910 (US); ZITZMANN, Nicole, Oxford OX1 4TL (GB); DWEK, Raymond, A., Oxford OX2 9AU (GB); BUTTERS, Terry, D., Oxford OX44 9BS (GB)
(74) Representative: HOFFMANN EITLE
(86) International application number: PCT/US2010/047498
(87) International publication number: WO 2011/028781

(56) References cited:
- EP-A2- 0 494 850
- WO-A1-95/22975
- WO-A1-2006/124676
- US-A1- 2007 275 998
- GU BAOHUA ET AL: "Antiviral profiles of novel iminocyclitol compounds against bovine viral diarrhea virus, West Nile virus, dengue virus and hepatitis B virus", ANTIVIRAL CHEMISTRY & CHEMOTHERAPY, BLACKWELL SCIENTIFIC PUBL., LONDON, GB, vol. 18, no. 1, 1 January 2007 (2007-01-01), pages 49-59, XP008120632, ISSN: 0956-3202
- RATNER L ET AL: "Inhibition of HIV and SIV infectivity by blockade of alpha-glucosidase activity", VIROLOGY, ACADEMIC PRESS,ORLANDO, US, vol. 181, no. 1, 1 March 1991 (1991-03-01) , pages 180-192, XP023057038, ISSN: 0042-6822, DOI: 10.1016/0042-6822(91)90483-R [retrieved on 1991-03-01]
- BUTTERS ET AL.: 'Imino sugar inhibitors for treating the lysosomal glycosphingolipidoses' GLYCOBIOLOGY vol. 15, no. 10, 2005, pages 43R - 52R, XP00812586

## Description

### RELATED APPLICATIONS

The present application claims priority to U.S. provisional application no. 61/272,252 filed September 4, 2009.

### FIELD

The present application relates to iminosugars and methods of treating viral infections with iminosugars and, in particular, to iminosugars for use in a method of treatment and/or prevention of viral infections caused by or associated with a virus belonging to the Poxviridae family.

### SUMMARY

One embodiment is a compound of the formula: 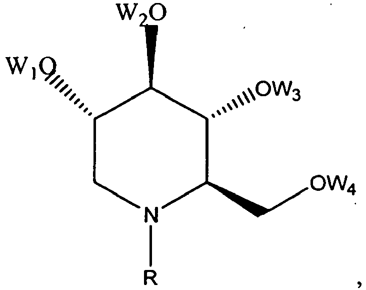 or a pharmaceutically acceptable salt thereof, for use in a method of treating or preventing in a subject a disease or condition caused by or associated with a virus belonging to the Poxviridae family, wherein R is either selected from substituted or unsubstituted oxaalkyl groups, substituted or unsubstituted alkyl groups, substituted or unsubstituted cycloalkyl groups, or substituted or unsubstituted aryl groups; or wherein R is 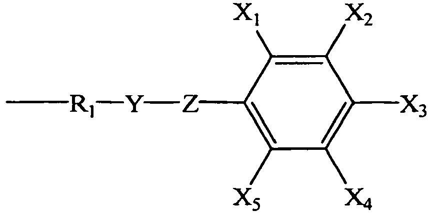
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

### DRAWINGS

Figures 1(A)-(E) present chemical formulas of the following iminosugars: A) *N*-Butyl deoxynojirimycin (NB-DNJ, UV-1); B) *N*-Nonyl deoxynojirimycin (NN-DNJ, UV-2); C) *N-*(7-Oxadecyl)deoxynojirimycin (N7-O-DNJ, UV-3); D) *N*-(9-Methoxynonyl) deoxynojirimycin (UV-4); E) *N*-(*N*-{4'-azido-2'-nitrophenyl}-6-aminohexyl)deoxynojirimycin (UV-5).

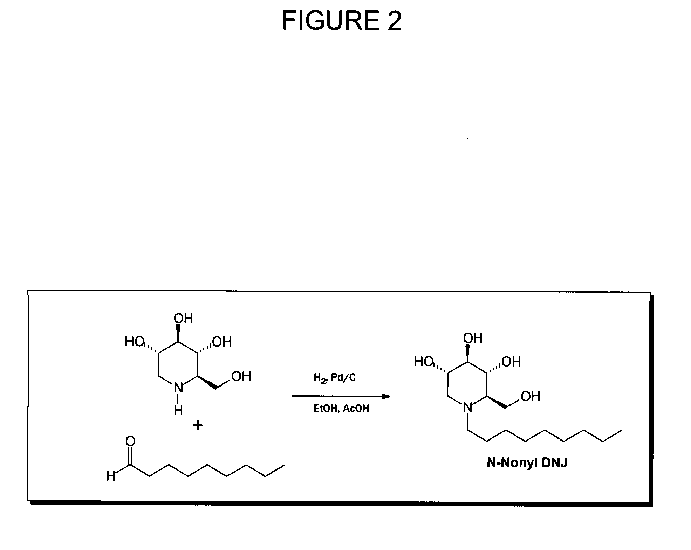

Figure 2 is a synthesis scheme for NN-DNJ.

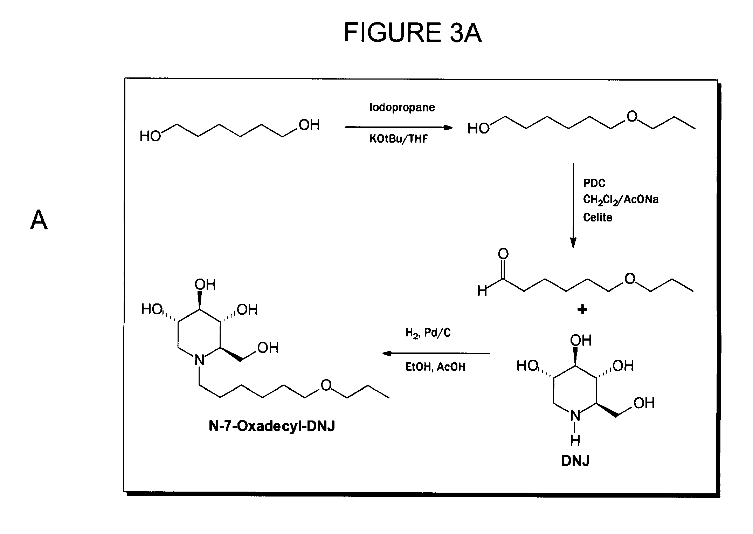

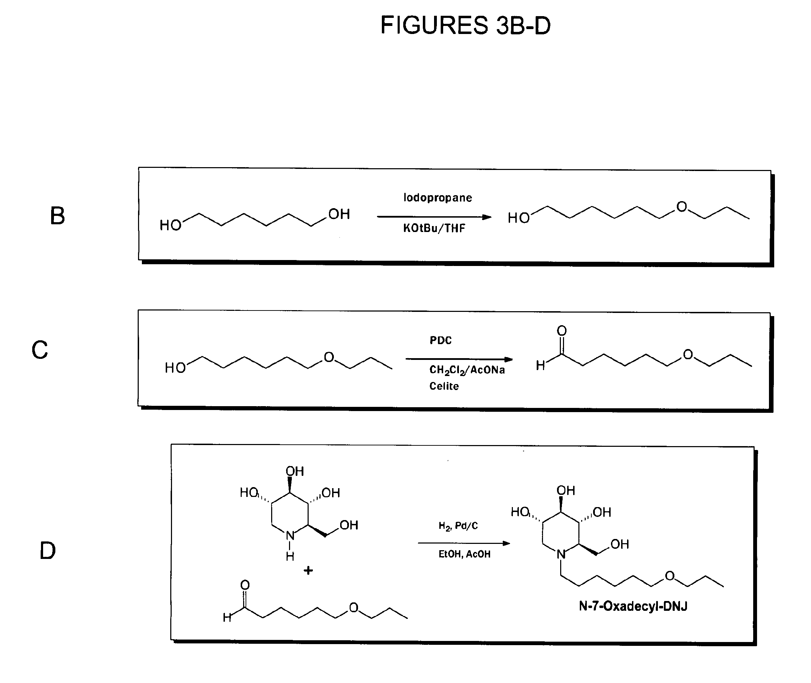

Figures 3A-D illustrate synthesis of N7-O-DNJ. In particular, Figure 3A shows a sequence of reactions leading to N7-O-DNJ; Figure 3B illustrates preparation of 6-propyloxy-1-hexanol; Figure 3C illustrates preparation of 6-propyloxy-1-hexanal; Figure 3D illustrates synthesis of N7-O-DNJ.
Figures 4A-C relate to synthesis of *N*-(9-Methoxynonyl) deoxynojirimycin. In particular,

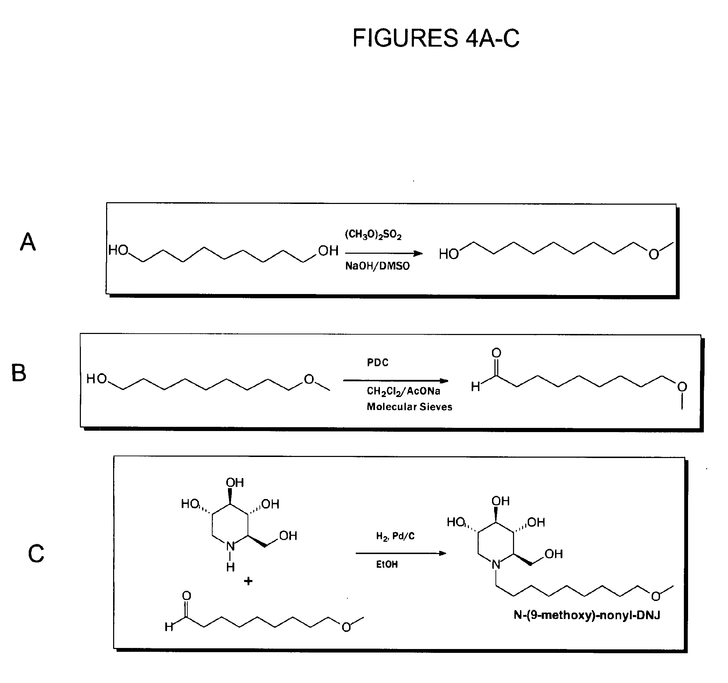

Figure 4A illustrates preparation of 9-methoxy-1-nonanol; Figure 4B illustrates preparation of 9-methoxy-1-nonanal; Figure 4C illustrates synthesis of *N*-(9-Methoxynonyl) deoxynojirimycin.

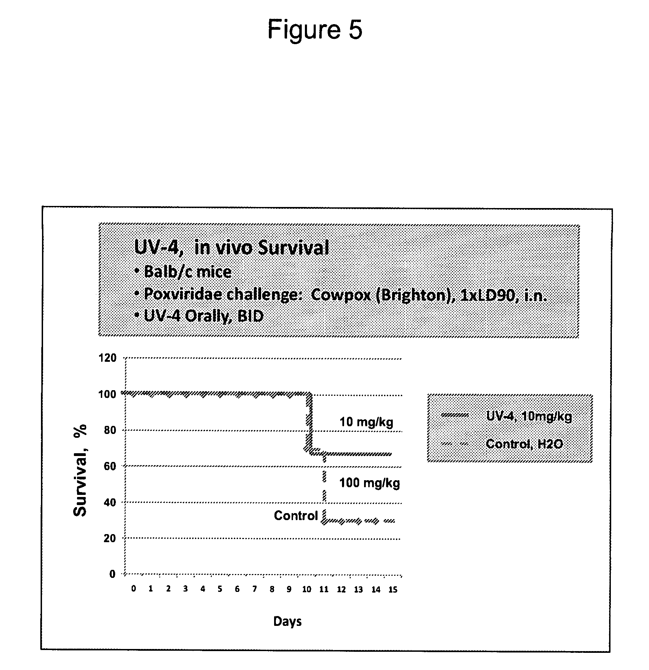

Figure 5 presents *in vivo* survival data for mice infected with cowpox virus.

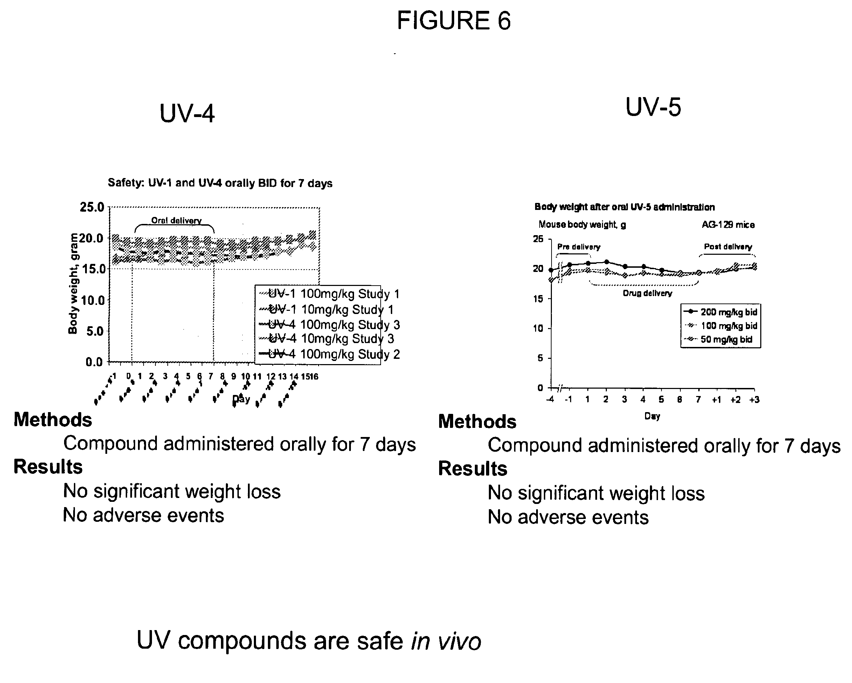

Figure 6 presents *in vivo* safety data for UV-4 and UV-5.

### DETAILED DESCRIPTION

### Related Applications

The following patent documents, may be useful for understanding the present disclosure:
1) US patent no. 6,545,021;
2) US patent no. 6,809,803;
3) US patent no. 6,689,759;
4) US patent no. 6,465,487;
5) US patent no. 5,622,972;
6) US patent application no. 12/656,992 filed February 22, 2010;
7) US patent application no. 12/656,993 filed February 22, 2010;
8) US patent application no. 12/813,882 filed June 11, 2010;
9) US patent provisional application no. 61/282,507 filed February 22, 2010;
10) US patent provisional application no. 61/272,252 filed September 4, 2009;
11) US provisional application no. 61/272,253 filed September 4, 2009;
12) US provisional application no. 61/272,254 filed September 4, 2009;
13) US provisional application no. 61/282,508 filed February 22, 2010;
14) US provisional application no. 61/353,935 filed June 11, 2010.

Some other relevant documents include:

WO 95/22975, which discloses a method for treating a mammal infected with respiratory syncytial virus (RSV) combrising administering to the mammal an RSV inhibitory effective amount of a compound or its pharmaceutically acceptable salt of formula (I), wherein R1 is alkyl, aryl, aralkyl, aroyl or acyl, and R2, R3, R4, and R5 are H or acyl.

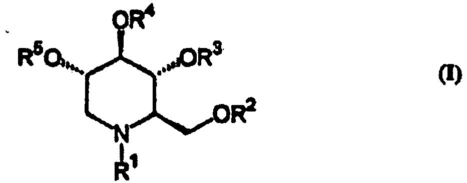

Antiviral Chemistry and Chemotherapy 18:49-59 discloses that the antiviral activity of iminocyclitol compounds with a deoxynojirimycin (DNJ) head group and either a straight chain alkyl or alkylcycloalkyl group attached to the nitrogen atom have been tested in vitro against multiple enveloped viruses.

Virology 181, 180-192 (1991) discloses that an inhibitor of alpha-glucosidases I and II, N-butyl-DNJ (NB-DNJ) retards HIV-I and SIVmac spread in lymphocytes and monocytes by diminishing virus infectivity, and also causes a reduction in syncytia formation between infected cells and uninfected lymphocytes. NB-DNJ retards envelope processing from the precursor form to the mature surface (SU) and transmembrane proteins in HIV-1 and SIVmac-infected cells, as well as in cells infected with vaccinia HIV-1 envelope recombinant virus. However, no significant reduction is seen in the amount of SU in released vvirus particles, though the virus particle-associated SU from NB-DNJ-treated cells has an altered electrophoretic mobility. In contrast, NB-DNJ had no effect on GAG protein synthesis and processing. These findings demonstrate a critical requirement for oligosaccharide processing by alpha-glucosidases I and II for HIV-I and SIVmac envelope processing and fusogenicity.

Finally, in EP 0494850, O-acylated derivatives of 1,5-dideoxy-1,5-imino-D-glucitol are disclosed that contain an N-alkyl or N-aroyl radical in which from one to four of the free hydroxyl groups are O-acylated with carboxylic alkanoyl radicals selected from the group consisting of ω,ω,ω-trifluoro alkanoyl having from three to eight carbon atoms, carboxylic cycloalkanoyl groups having from four to eight carbon atoms and carboxylic acyclic alkanoyl groups having from two to ten carbon atoms, wherein the N-aroyl radical is selected from the group consisting of p-decylbenzoyl, 3-(p-chlorophenoxy)-propanoyl, 2-(acetyloxy)benzoyl, [1,1' -biphenyl]-4-ylcarbonyl, 2-thiopheneacetyl, trans-3-furanacryloyl, 3-methoxyphenylacetyl and 3-(trifluoromethyl)benzoyl, and wherein the N-alkyl contains from one to fourteen carbon atoms, provided that when N-alkyl contains from one to five carbon atoms the O-acylated groups are ω,ω,ω-trifluoro alkanoyl or carboxylic cycloalkanoyl.

### Definition of terms

Unless otherwise specified, "a" or "an" means "one or more."

As used herein, the term "viral infection" describes a diseased state, in which a virus invades a healthy cell, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating or preventing viral infection" means to inhibit the replication of the particular virus, to inhibit viral transmission, or to prevent the virus from establishing itself in its host, and to ameliorate or alleviate the symptoms of the disease caused by the viral infection. The treatment is considered therapeutic if there is a reduction in viral load, decrease in mortality and/or morbidity.

IC50 or IC90 (inhibitory concentration 50 or 90) is a concentration of a therapeutic agent, such as an iminosugar, used to achieve 50% or 90% reduction of viral load, respectively.

### Disclosure

The present inventors discovered that certain iminosugars, such as deoxynojirimycin derivatives, may be effective against viruses belonging to the Poxviridae family.

In particular, such iminosugars may be useful in a method of treating or preventing a disease or condition caused by or associated with a virus belonging to the Poxviridae family.

The Poxviridae family includes the Chordopoxviridae subfamily and the Entomopoxviridae subfamily. The Chordopoxviridae subfamily includes Orthopox genus, Parapox genus; Aviropox genus; Capripoxvirus genus; Leporipoxvirus genus; Suipoxvirus genus; Molluscipoxvirus genus and Yatapox genus. The Entomopoxviridae subfamily includes Entomopoxviruses A, B and C. Viruses of orthopox, parapox, yatapox and molluscipox genera may infect humans.

Viruses belonging to the Orthopoxvirus genus of the Poxviridae family, i.e., orthopoxviruses, include Buffalopox virus; Camelpox virus; Cowpox virus; Ectromelia virus; Monkeypox virus; Rabbitpox virus; Raccoonpox virus; Sealpox virus; Skunkpox virus; Taterapox virus; Uasin Gishu disease virus; Vaccinia virus; Variola virus; and Volepox virus.

Diseases caused by or associated with orthopoxviruses include Buffalopox; Camelpox; Cowpox; Mousepox (cause by Ectromelia virus); Monkeypox; Rabbitpox, also known as Green Rabbit Syndrome; Raccoonpox; Sealpox; Skunkpox; Taterapox; Uasin Gishu disease; Smallpox; and Volepox.

Viruses belonging to the Parapox genus of the Poxviridae family, i.e. parapoxviruses, include orf virus, pseudocowpox and bovine papular stomatitis virus.

Diseases caused by or associated with parapoxviruses include orf, pseudocowpox and bovine papular stomatitis.

Viruses belonging to the Yatapox genus of the Poxviridae family, i.e. yatapoxviruses, include tanapox virus and yaba monkey tumor virus.

Molluscum contagiosum virus is an example of a molluscipox virus, i.e. a virus belonging to the Molluscipox genus of the Poxviridae family.

In many embodiments, the iminosugar may be N-substituted deoxynojirimycin. In some embodiments, as the N-substituted deoxynojirimycin may be a compound of the following formula: 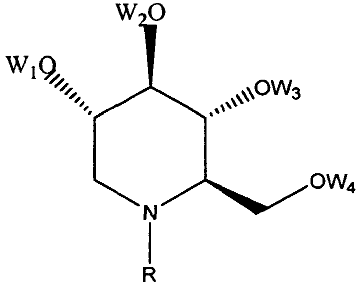 where W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

In some embodiments, R may be selected from substituted or unsubstituted alkyl groups, substituted or unsubstituted cycloalkyl groups, substituted or unsubstituted aryl groups, or substituted or unsubstituted oxaalkyl groups.

In some embodiments, R may be substituted or unsubstituted alkyl groups and/or substituted or unsubstituted oxaalkyl groups comprise from 1 to 16 carbon atoms, from 4 to 12 carbon atoms or from 8 to 10 carbon atoms. The term "oxaalkyl" refers to an alkyl derivative, which may contain from 1 to 5 or from 1 to 3 or from 1 to 2 oxygen atoms. The term "oxaalkyl" includes hydroxyterminated and methoxyterminated alkyl derivatives.

In some embodiments, R may be selected from, but is not limited
to -(CH₂)₆OCH₃, -(CH₂)₆OCH₂CH₃, -(CH₂)₆O(CH₂)₂CH₃, -(CH₂)₆O(CH₂)₃CH₃, -(CH₂)₂O(C H₂)₅CH₃, -(CH₂)₂O(CH₂)₆CH₃,;-(CH₂)₂O(CH₂)₇CH₃; -(CH₂)₉-OH; -(CH₂)₉OCH₃.

In some embodiments, R may be branched or unbranched, substituted or unsubstituted alkyl group. In certain embodiments, the alkyl group may be a long chain alkyl group, which may be C6-C20 alkyl group; C8-C16 alkyl group; or C8-C 10 alkyl group. In some embodiments, R may be a long chain oxaalkyl group, i.e. a long chain alkyl group, which may contain from 1 to 5 or from 1 to 3 or from 1 to 2 oxygen atoms.

In some embodiments, R may have the following formula 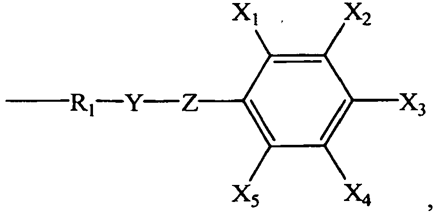 where R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl.

In some embodiments, Z is NH and R₁-Y is a substituted or unsubstituted alkyl group, such as C2-C20 alkyl group or C4-C12 alkyl group or C4-C 10 alkyl group.

In some embodiments, X₁ is NO₂ and X₃ is N₃. In some embodiments, each of X₂, X₄ and X₅ is hydrogen.

In some embodiments, the iminosugar may be a DNJ derivative disclosed in U.S. Patent application publication no. 2007/0275998.

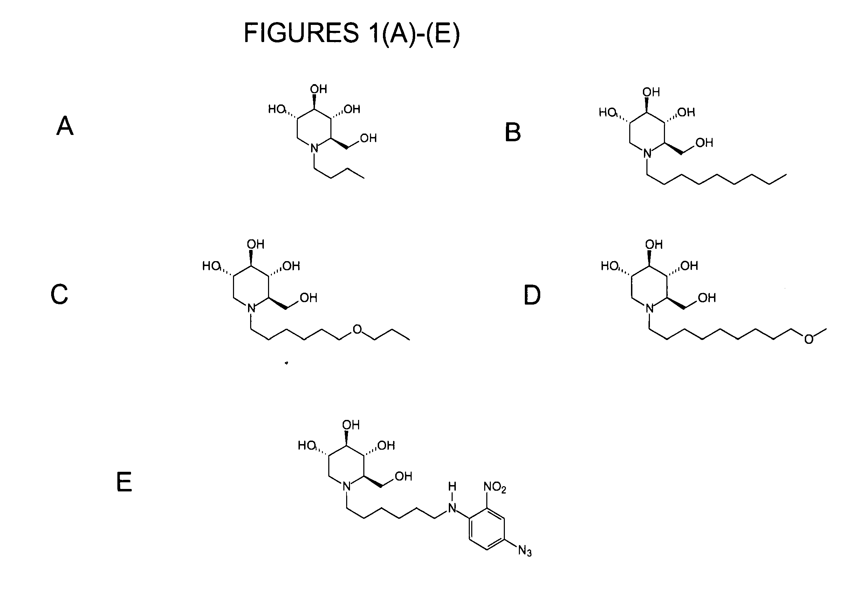

In some embodiments, the iminosugar may be one of the compounds presented in Figure 1. Methods of synthesizing deoxynojirimycin derivatives are disclosed, for example, in U.S.

Patent Nos. 5,622,972, 5,200,523, 5,043,273, 4,994,572, 4,246,345, 4,266,025, 4,405,714, and 4,806,650 and U.S. Patent application publication no. 2007/0275998.

In some embodiments, the iminosugar may be in a form of a salt derived from an inorganic or organic acid. Pharmaceutically acceptable salts and methods for preparing salt forms are disclosed, for example, in Berge et al. (J. Pharm. Sci. 66:1-18, 1977). Examples of appropriate salts include but are not limited to the following salts: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate, and undecanoate.

In some embodiments, the iminosugar may also used in a form of a prodrug. Prodrugs of DNJ derivatives, such as the 6-phosphorylated DNJ derivatives, are disclosed in U.S. Patents nos. 5,043,273 and 5,103,008.

In some embodiments, the iminosugar may be used as a part of a composition, which further comprises a pharmaceutically acceptable carrier and/ or a component useful for delivering the composition to an animal. Numerous pharmaceutically acceptable carriers useful for delivering the compositions to a human and components useful for delivering the composition to other animals such as cattle are known in the art. Addition of such carriers and components to the composition of the invention is well within the level of ordinary skill in the art.

In some embodiments, the pharmaceutical composition may consist essentially of *N-*substituted deoxynojirimycin, which may mean that the *N*-substituted deoxynojirimycin is the only active ingredient in the composition.

Yet in some embodiments, N-substituted deoxynojirimycin may be administered with one or more additional antiviral compounds.

In some embodiments, the iminosugar may be used in a liposome composition, such as those disclosed in US publications nos. 2008/0138351 and 2009/0252785 as well as in US application No. 12/732630 filed March 26, 2010.

The iminosugar, such as a DNJ derivative, may be administered to an animal affected by a virus. The iminosugar may inhibit morphogenesis of the virus, or it may treat the individual. The treatment may reduce, abate, or diminish the virus infection in the animal.

Animals that may be infected with poxviruses include mammals including bovids, such as buffalos, sheep, goats and cattle (cows); camels; rodents, such as mice, voles, and gerbils; leporids, such as rabbits and hares; raccoons; seals; skunks; equines, including horses; primates, including monkeys and humans.

The amount of iminosugar administered to an animal to the methods of the invention may be an amount effective to inhibit the morphogenesis of a poxvirus. The term "inhibit" as used herein may refer to the detectable reduction and/or elimination of a biological activity exhibited in the absence of the iminosugar. The term "effective amount" may refer to that amount of the iminosugar necessary to achieve the indicated effect. The term "treatment" as used herein may refer to reducing or alleviating symptoms in a subject, preventing symptoms from worsening or progressing, inhibition or elimination of the causative agent, or prevention of the infection or disorder related to the poxvirus in a subject who is free therefrom.

Thus, for example, treatment of the disease caused by or associated with a virus may include destruction of the infecting agent, inhibition of or interference with its growth or maturation, and neutralization of its pathological effects. The amount of the iminosugar which may be administered to the animal is preferably an amount that does not induce any toxic effects which outweigh the advantages which accompany its administration.

Actual dosage levels of active ingredients in the pharmaceutical compositions may vary so as to administer an amount of the active compound(s) that is effective to achieve the desired therapeutic response for a particular patient.

The selected dose level may depend on the activity of the iminosugar, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient may depend on a variety of factors, including the body weight, general health, diet, time and route of administration and combination with other therapeutic agents and the severity of the condition or disease being treated. In some embodiments, the adult human daily dosage may range from between about one microgram to about one gram, or from between about 10 mg and 100 mg, of the iminosugar per 10 kilogram body weight. In some embodiments, a total daily dose may be from 0.1 mg/kg body weight to 100 mg/kg body weight or from 1 mg/kg body weight to 60 mg/kg body weight or from 2 mg/kg body weight to 50 mg/kg body weight or from 3 mg/kg body weight to 30 mg/kg body weight. The daily dose may be administered over one or more administering events over day. For example, in some embodiments, the daily dose may be distributed over two (BID) administering events per day, three administering events per day (TID) or four administering events (QID). In certain embodiments, a single administering event dose ranging from 1 mg/kg body weight to 10 mg/kg body weight may be administered BID or TID to a human making a total daily dose from 2 mg/kg body weight to 20 mg/kg body weight or from 3 mg/kg body weight to 30 mg/kg body weight. Of course, the amount of the iminosugar which should be administered to an animal may depend upon numerous factors well understood by one of skill in the art, such as the molecular weight of the iminosugar and the route of administration.

Pharmaceutical compositions that are useful in the methods of the invention may be administered systemically in oral solid formulations, ophthalmic, suppository, aerosol, topical or other similar formulations. For example, it may be in the physical form of a powder, tablet, capsule, lozenge, gel, solution, suspension, syrup, or the like. In addition to the iminosugar, such pharmaceutical compositions may contain pharmaceutically-acceptable carriers and other ingredients known to enhance and facilitate drug administration. Other possible formulations, such as nanoparticles, liposomes, resealed erythrocytes, and immunologically based systems may also be used to administer the iminosugar. Such pharmaceutical compositions may be administered by a number of routes. The term "parenteral" used herein includes subcutaneous, intravenous, intraarterial, intrathecal, and injection and infusion techniques, without limitation. By way of example, the pharmaceutical compositions may be administered orally, topically, parenterally, systemically, or by a pulmonary route.

These compositions may be administered in a single dose or in multiple doses which are administered at different times. Because the inhibitory effect of the composition upon a poxvirus may persist, the dosing regimen may be adjusted such that virus propagation is retarded while the host cell is minimally effected. By way of example, an animal may be administered a dose of the composition of the invention once per week, whereby virus propagation is retarded for the entire week, while host cell functions are inhibited only for a short period once per week.

Embodiments described herein are further illustrated by, though in no way limited to, the following working examples.

### Working Examples

### 1. Synthesis of N-Nonyl DNJ

**Table 1. Materials for NN-DNJ synthesis**

| Name | Amount |
|---|---|
| DNJ | 500 mg |
| Nonanal | 530 mg |
| Ethanol | 100 mL |
| AcOH | 0.5 mL |
| Pd/C | 500 mg |

Procedure: A 50-mL, one-necked, round-bottom flask equipped with a magnetic stirrer was charged with DNJ (500 mg), ethanol (100 mL), nonanal (530 mg), and acetic acid (0.5 mL) at room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-25%) was used to elute the product from the column. All fractions containing the desired product were combined, and concentrated *in vacuo* to give the pure product (420mg). Completion of the reaction was monitored by thin layer chromatography (TLC) using a thin layer silica gel plate; eluent; methanol : dichloromethane = 1:2

### 2. Synthesis of N-7-Oxadecyl DNJ

### 2a. Synthesis of 6-propyloxy-1-hexanol

**Table 2. Materials for synthesis of 6-propyloxy-1-hexanol**

| Name | Amount |
|---|---|
| 1,6-hexanediol | 6.00 g |
| 1-Iodopropane | 8.63 g |
| Potassium tert-butoxide | 5.413 mg |
| THF | 140 mL |

Procedure: a 500-mL, one-necked, round-bottom flask equipped with a magnetic stirrer was charged with 1,6-hexanediol (6.00 g), potassium tert-butoxide (5.413 g) at room temperature. The reaction mixture was stirred for one hour, and then 1-iodopropane (8.63 g) was added. The reaction mixture was heated to 70-80 °C and stirred overnight. The progress of reaction was monitored by TLC (Note 1). After completion of the reaction, water was added to the reaction mixture, and extracted with ethyl acetate (2 x 100 mL). The combined organic layers were concentrated *in vacuo* to get the crude product. The crude product was dissolved in dichloromethane and washed with water, and then brine, dried over sodium sulfate. The organic layer was concentrated *in vacuo* to get the crude product. The crude product was purified by column chromatography using 230-400 mesh silica gel. A solvent gradient of ethyl acetate in hexanes (10-45%) was used to elute the product from the column. All fractions containing the desired pure product were combined and concentrated *in vacuo* to give pure 6-propyloxy-1-hexanol (lot D-1029-048, 1.9 g, 25%) Completion of the reaction was monitored by thin layer chromatography (TLC); (eluent: 60% ethyl acetate in hexanes).

### 2b. Preparation of 6-propyloxy-1-hexanal

**Table 3. Materials for preparation of 6-propyloxy-1-hexanal**

| Name | Amount |
|---|---|
| 6-Propyloxy-1-hexanol | 1.00 g |
| PDC | 4.70 g |
| Celite | 1.00 g |
| NaOAc | 100 mg |
| CH₂Cl₂ | 10 mL |

Procedure: a 50-mL, one-necked, round-bottom flask equipped with a magnetic stirrer was charged with 6-propyloxy-1-hexanol (1.0 g), PDC (4.7 g), dichloromethane (10 mL), Celite (1.0 g), and sodium acetate (100 mg). The reaction mixture was stirred at room temperature under nitrogen for 5 minutes. PDC (4.70 g) was added to the reaction mixture, and stirred overnight. The progress of reaction was monitored by TLC (Note 1). After completion of the reaction, the reaction mixture was directly loaded on the column (230-400 mesh silica gel). A solvent gradient of dichloromethane in ethyl acetate (10-20%) was used to elute the product from the column. All fractions containing the desired pure product were combined and concentrated *in vacuo* to give pure 6-propyloxy-1-hexanal (lot D-1029-050, 710 mg, 71%). Completion of the reaction was monitored by thin layer chromatography (TLC); (eluent: 60% ethyl acetate in hexanes).

### 2c Synthesis of N-7-Oxadecyl-DNJ

**Table 4. Materials for Synthesis of N-7-Oxadecyl-DNJ**

| Name | Amount |
|---|---|
| DNJ | 500 mg |
| 6-Propyloxy-1-hexanal | 585 mg |
| Pd/C | 125 mg |
| Ethanol | 15 mL |
| Acetic acid | mL |

Procedure: a 50-mL, one-necked, round-bottom flask equipped with a magnetic stirrer was charged with DNJ (500 mg), ethanol (15 mL), 6-propyloxy-1-hexanal (585 mg), and acetic acid (0.1mL) t room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-40%) was used to elute the product from the column. All fractions containing the desired product were combined, and concentrated *in vacuo* to give the pure product. (Lot: D-1029-052 (840 mg). Completion of the reaction was monitored by thin layer chromatography (TLC); (eluent: 50% methanol in dichloromethane).

### 3. Synthesis of N-(9-methoxy)-nonyl DNJ

### 3a Preparation of 9-methoxy-1-nonanol

**Table 5. Materials for preparation of 9-methoxy-1-nonanol**

| Name | Amount |
|---|---|
| 1,9-nonanediol | 10.0 g |
| Dimethyl sulfate | 41.39 g |
| Sodium hydroxide | 5.0g |
| DMSO | 100 mL |

Procedure: a 500-mL, one-necked, round-bottom flask equipped with a magnetic stirrer and stir bar was charged with 1,9-nonanediol (10.00 g, 62.3 mmol) in dimethyl sulfoxide (100 mL) and H₂O (100 mL). To this was added slowly a solution of sodium hydroxide (5.0 g, 125.0 mmol) in H₂O (10 mL) at room temperature. During addition of sodium hydroxide the reaction mixture generated heat and the temperature rose to ∼40 °C. The mixture was stirred for one hour, and then dimethyl sulfate (16.52 g, 131 mmol) was added in four portions while maintaining the temperature of the reaction mixture at ~ 40°C. The reaction mixture was stirred at room temperature overnight. Progress of the reaction was monitored by TLC (Note 1). TLC monitoring indicated that the reaction was 25 % conversion. At this stage additional dimethyl sulfate (24.78g, 196.44 mmol) was added and the resulting mixture was stirred at room temperature for an additional 24 h. After completion of the reaction, sodium hydroxide (10% solution in water) was added to the reaction mixture to adjust the pH of the solution to 11-13. The mixture was stirred at room temperature for 2 h and extracted with dichloromethane (3 x 100 mL). The combined organic layers were washed with H₂O (200 mL), brine (150 mL), dried over anhydrous sodium sulfate (20 g), filtered and concentrated *in vacuo* to obtain a crude product (14 g). The crude product was purified by column chromatography using 250-400 mesh silica gel. A solvent gradient of ethyl acetate in hexanes (10-50%) was used to elute the product from the column. All fractions containing the desired pure product were combined and concentrated *in vacuo* to give pure 9-methoxy-1-nonanol (lot D-1027-155, 2.38 g, 21.9 %). Completion of the reaction was monitored by thin layer chromatography (TLC) using a thin layer silica gel plate; eluent: 60% ethyl acetate in hexanes.

### 3b Preparation of 9-methoxy-1-nonanal

**Table 6. Materials for preparation of 9-methoxy-1-nonanal**

| Name | Amount |
|---|---|
| 9-methoxy-1-nonanol | 1.0 g |
| PDC | 4.7 g |
| Molecular sieves, 3A | 1.0 g |
| NaOAc | 0.1g |
| CH₂Cl₂ | 10 mL |

Procedure: a 50-mL, one-necked, round-bottom flask equipped with a magnetic stirrer and stir bar was charged with 9-methoxy-nonanol (1.0 g, 5.9 mmol), dichloromethane (10 mL), molecular sieves (1.0 g, 3A), sodium acetate (0.1 g) at room temperature. The reaction mixture was stirred at room temperature under nitrogen for 5 minutes. The reaction mixture was charged with pyridinium dichromate (4.7 g, 12.5 mmol) and stirred overnight. The progress of reaction was monitored by TLC (Note 1). After completion of the reaction, the reaction mixture was filtered through a bed of silica gel (∼15 g). The filtrate was evaporated in *vacuo* to obtain a crude compound. This was purified by column chromatography using silica gel column (250-400 mesh, 40 g). A solvent gradient of ethyl acetate in hexane (10-50%) was used to elute the product from the column. All fractions containing the desired pure product were combined and concentrated *in vacuo* to give pure 9-methoxy-nonanal (lot D-1027-156, 553 mg, 54.4%). Completion of the reaction was monitored by thin layer chromatography (TLC) using a thin layer silica gel plate; eluent: 60% ethyl acetate in hexanes.

### 3c Synthesis ofN-(9-methoxy)-nonyl DNJ

**Table 7. Materials for synthesis ofN-(9-methoxy)-nonyl DNJ**

| Name | Amount |
|---|---|
| DNJ | 300 mg |
| 9-methoxy-1-nonanal | 476 mg |
| Pd/C | 200 mg |
| Ethanol | 20 mL |

Procedure: a 50-mL, two-necked, round-bottom flask equipped with magnetic stirrer and a stir bar was charged with DNJ (300 mg, 1.84 mmol), ethanol (20 mL), 9-methoxy-1-nonanal (476 mg, 2.76 mmol) at room temperature. The reaction mixture was stirred for 5-10 minutes under nitrogen and Pd/C was added at room temperature. The reaction mixture was evacuated and was replaced by hydrogen gas using a balloon. This process was repeated three times and then reaction mixture was stirred under atmospheric hydrogen at room temperature. The progress of reaction was monitored by TLC (Note 1). The reaction mixture was filtered through a bed of Celite and was washed with ethanol (20 mL). The filtrate was concentrated *in vacuo* to get a crude product. The crude product was purified by column chromatography using 250-400 mesh silica gel (20 g). A solvent gradient of methanol in ethyl acetate (5-25%) was used to elute the product from the column. All fractions containing the desired pure product were combined, and concentrated *in vacuo* to give an off white solid. The solid was triturated in ethyl acetate (20 mL), filtered and dried in high vacuum to give a white solid [lot: D-1027-158 (165.3 mg, 28.1%). Completion of the reaction was monitored by thin layer chromatography (TLC) using a thin layer silica gel plate; eluent: 50% methanol in dichloromethane.

### 4. Effects of iminosugars against Vaccinia Virus

Table 7 provides data for inhibition of infectivity of Vaccinia virus for NB-DNJ (UV-1), NN-DNJ (UV-2), N7-O-DNJ (UV-3), N9-DNJ (UV-4) and NAP-DNJ (UV-5).

**Table 7.**

| Compound | IC50, µM |
|---|---|
| UV-1 | 90 |
| UV-2 | 21 |
| UV-3 | 7 |
| UV-4 | 59 |
| UV-5 | 3 |

Procedure. The compounds were screened for inhibition of generation of infectious virus was conducted on the UV compounds at concentrations from 4 µM up to 250 µM. The orthopoxvirus Vaccinia NYCBOH strain was evaluated for virus inhibition. BSC-40 cells (vervet monkey kidney epithelial cell line) obtained from American Type Culture Collection (ATCC, Manassas, Virginia). Cells were cultured in 1x modified Eagle medium (MEM, Gibco), supplemented with 5% fetal bovine serum, 2 mM L-glutamine, 100 U/ml penicillin, 100 µg/ml streptomycin in cell culture treated 24-well flat bottom plates at 37°C in a 5% CO2 incubator for 24 hr or until 80% confluent prior to assay. Cells were pretreated with compounds in a final concentration of 0.5% DMSO for 1 hr followed addition of virus inoculums in EMEM with 5% FBS. Three days later virus containing supernatants were collected and 10 fold dilutions of virus-containing supernatants was done in a virus plaque assay. To titer, 12-well plates with 80% confluent BSC-40 cells in growth medium were used. Viral supernatant were diluted from 10⁻³ to 10⁻⁸ and added to the cells and incubated at 37°C for 1 hour with shaking every 5-10 minutes. Viral infection medium were aspirated and replace with 1mL pre-warmed 2% low-melt agarose mixed 1:1 with 2X MEM (5% fetal calf serum final concentration) and incubated at 37°C, 5% CO₂ for 2 days followed by plaque visualization by neutral red staining.

### EXAMPLE 5

The study assessed the efficacy of the iminosugar compound, UV-4, in promoting survival of mice challenged with Cowpox Brighton. This compound was previously tested in both in vitro (CC50 of 125 to >2,000uM) and in vivo (no weight loss or adverse effects observed in multiple mouse studies) and shown it possesses low toxicity. In this study, the compound was administered as a free drug dissolved in water. The UV-4 compound was given by the oral route (2x per day intragastric via oral gavage - IG) for a total number of 10 days after the start of the compound dosing. Study animals were infected intranasally with cowpox brighton with -1 LD90 (1.00e6 pfu/mouse) 1 hour before the first UV-4 dose.

### Methods:

Infection: 4-6 week old female BALB/C mice were anesthetized with isofluorene prior to intranasal inoculation with 100uL Cowpox Brighton (Where did you obtain this strain? Is it publically available?) at a concentration of 1xLD90.

Dosing: 2X per day mice (n=10) were orally gavaged with 100ul of the compound dilution (prepared in H2O). Treatments lasted for 10 days.

### Results:

**Table 8.**

| Days post infection. | Control + H₂O, % | UV-4 0.2mg, % |
|---|---|---|
| 0 | 100 | 100 |
| 10 | 70 | 100 |
| 11 | 30 | 70 |

Figure 5 shows survival data for mice that were infected with a 1xLD90 dose of cowpox brighton and dosed 3x per day for 10 days with either water (control group) or UV-4 (treated group). Table 8 shows a percentage of surviving mice in a) the control group treated with water and b) the group treated with UV-4 on days indicated in the left column. Each of the control and treated groups included 10 mice.

Kaplan-Meier analysis of the control and UV-4 treated groups. Log-rank (Mantel Cox) Analysis indicating p values between the groups. A p value of <0.05 indicates significance. Mice P-value for UV-4 0.2 mg is 0.046.

### EXAMPLE 6

### Iminosugar Safety Study

Methods and Discussion: BALB/c and C57/B1/6 mice were given oral suspensions of UV-1, UV-4, UV-5, twice a day for seven days, in 100ul per mouse at 100 and 10 mg/kg (2mg and 0.2 mg/mouse, respectively) 8 hours apart for 7 days, and then monitored for weight loss and general health. After seven days of treatment, the mice did not show any significant signs of weight loss compared to the "vehicle only" control. The results of these experiments are in Figure 6.

When the BALB/c mice were treated with UV-5 at the highest concentration, they displayed signs of diarrhea, red urine, and a ruffled appearance although they did not show signs of weight loss. The C57/B1/6 mice displayed these same symptoms but without the ruffled look. These symptoms promptly ceased when treatment was done, and by day 11 (day 4 post compound treatment) the BALB/c mice in these groups looked very healthy.

Conclusions: These compounds have shown to be relatively non-toxic in this mouse model and these concentrations of compound are deemed safe.

## Claims

1. A compound of the formula: 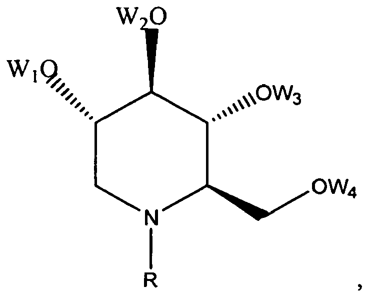 or a pharmaceutically acceptable salt thereof, for use in a method of treating or preventing in a subject a disease or condition caused by or associated with a virus belonging to the Poxviridae family, wherein R is either selected from substituted or unsubstituted oxaalkyl groups, substituted or unsubstituted alkyl groups, substituted or unsubstituted cycloalkyl groups, or substituted or unsubstituted aryl groups; or wherein R is 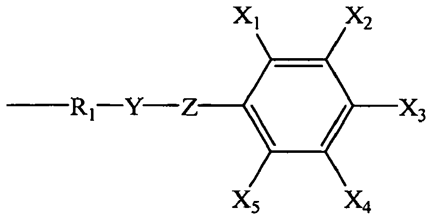
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

2. The compound for use of claim 1, wherein each of W₁, W₂, W₃ and W₄ is hydrogen.

3. The compound for use of claim 1 or claim 2, wherein R is selected from substituted or unsubstituted oxaalkyl groups, substituted or unsubstituted alkyl groups, substituted or unsubstituted cycloalkyl groups, and substituted or unsubstituted aryl groups.

4. The compound for use of any one of claims 1-3, wherein R is C6-C12 oxaalkyl or alkyl group.

5. The compound for use of claim 4, wherein R is C8-C 10 oxaalkyl or alkyl group.

6. The compound for use of claim 1, wherein said compound is (i) N-(9-Methoxynonyl)deoxynojirimycin or a pharmaceutically acceptable salt thereof; (ii) N-(N-{4'-azido-2'-nitrophenyl}-6-aminohexyl)deoxynojirimycin or a pharmaceutically acceptable salt thereof; (iii) N-(7-oxadecyl)deoxynojirimycin or a pharmaceutically acceptable salt thereof or (iv) N-nonyl deoxynojirimycin or a pharmaceutically acceptable salt thereof.

7. The compound for use of claim 1 or claim 2, wherein R is

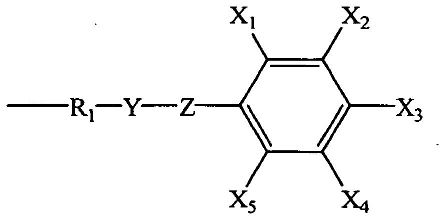

8. The compound for use of claim 7, wherein X₁ is NO₂ and X₃ is N₃.

9. The compound for use of claim 7, wherein each of X₂, X₄ and X₅ is hydrogen.

10. The compound for use of any one of claims 1-9, wherein the subject is a mammal.

11. The compound for use according to claim 10, wherein the subject is a human being.

12. The compound for use of any one of claims 1-9, wherein the disease or condition is smallpox.

13. The compound for use of any one of claims 1-12, wherein the virus belongs to the Orthopoxvirus family.

14. The compound for use of any one of claims 1-13, wherein the virus is Variola virus.

15. The compound for use of any one of claims 1-11 and 13, wherein the virus is Vaccinia virus or a cowpox virus.

## Patentansprüche

1. Verbindung der Formel: 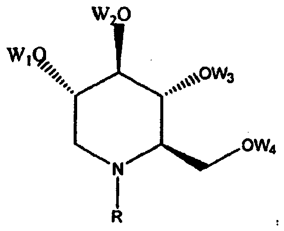 oder ein pharmazeutisch annehmbares Salz davon zur Verwendung bei einem Verfahren zur Behandlung oder Verhinderung einer Krankheit oder eines Zustands, die bzw. der von einem Virus aus der Familie Poxviridae verursacht wurde oder mit diesem assoziiert ist, bei einem Probanden, wobei R entweder aus substituierten oder unsubstituierten Oxaalkylgruppen, substituierten oder unsubstituierten Alkylgruppen, substituierten oder unsubstituierten Cycloalkylgruppen oder substituierten oder unsubstituierten Arylgruppen ausgewählt ist;
oder wobei R 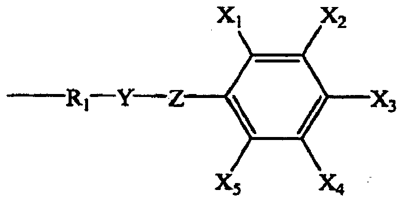 ist,
R₁ eine substituierte oder unsubstituierte Alkylgruppe ist;
X₁₋₅ unabhängig aus H, NO₂, N₃ oder NH₂ ausgewählt sind;
Y nicht vorhanden ist oder eine substituierte oder unsubstituierte von Carbonyl verschiedene C₁-Alkylgruppe ist; und
Z aus einer Bindung oder NH ausgewählt ist; mit der Maßgabe, dass, wenn Z eine Bindung ist, Y nicht vorhanden ist, und mit der Maßgabe, dass, wenn Z NH ist, Y eine substituierte oder unsubstituierte von Carbonyl verschiedene C₁-Alkylgruppe ist; und wobei W₁₋₄ unabhängig voneinander aus Wasserstoff, substituierten oder unsubstituierten Alkylgruppen, substituierten oder unsubstituierten Halogenalkylgruppen, substituierten oder unsubstituierten Alkanoylgruppen, substituierten oder unsubstituierten Aroylgruppen oder substituierten oder unsubstituierten Halo-alkanoylgruppen ausgewählt sind.

2. Verbindung zur Verwendung nach Anspruch 1, wobei jedes von W₁, W₂, W₃ und W₄ Wasserstoff ist.

3. Verbindung zur Verwendung nach Anspruch 1 oder Anspruch 2, wobei R aus substituierten oder unsubstituierten Oxaalkylgruppen, substituierten oder unsubstituierten Alkylgruppen, substituierten oder unsubstituierten Cycloalkylgruppen und substituierten oder unsubstituierten Arylgruppen ausgewählt ist.

4. Verbindung zur Verwendung nach einem der Ansprüche 1-3, wobei R C6-C12-oxaalkyl- oder -alkylgruppe ist.

5. Verbindung zur Verwendung nach Anspruch 4, wobei R C8-C10-oxaalkyl- oder -alkylgruppe ist.

6. Verbindung zur Verwendung nach Anspruch 1, wobei die Verbindung (i) N-(9-Methoxynonyl)deoxynojirimycin oder ein pharmazeutisch annehmbares Salz davon; (ii) N-(N-{4'-Azido-2'-nitrophenyl}-6-aminohexyl)deoxynojirimycin oder ein pharmazeutisch annehmbares Salz davon; (iii) N-(7-Oxadecyl)desoxynojirimycin oder ein pharmazeutisch annehmbares Salz davon oder (iv) N-Nonyldesoxynojirimycin oder ein pharmazeutisch annehmbares Salz davon ist.

7. Verbindung zur Verwendung nach Anspruch 1 oder Anspruch 2, wobei R 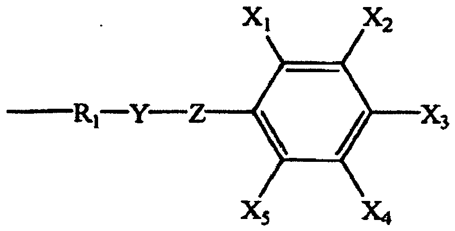 ist.

8. Verbindung zur Verwendung nach Anspruch 7, wobei X₁ NO₂ ist und X₃ N₃ ist.

9. Verbindung zur Verwendung nach Anspruch 7, wobei jedes von X₂, X₄ und X₅ Wasserstoff ist.

10. Verbindung zur Verwendung nach einem der Ansprüche 1-9, wobei der Proband ein Säuger ist.

11. Verbindung zur Verwendung nach Anspruch 10, wobei der Proband ein Mensch ist.

12. Verbindung zur Verwendung nach einem der Ansprüche 1-9, wobei die Erkrankung oder der Zustand Pocken ist.

13. Verbindung zur Verwendung nach einem der Ansprüche 1-12, wobei das Virus zu der Orthopoxvirus-Familie gehört.

14. Verbindung zur Verwendung nach einem der Ansprüche 1-13, wobei das Virus Variola-Virus ist.

15. Verbindung zur Verwendung nach einem der Ansprüche 1-11 und 13, wobei das Virus Vaccinia-Virus oder ein Kuhpocken-Virus ist.

## Revendications

1. Composé de formule : 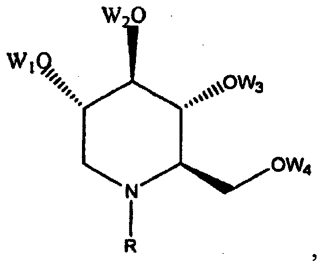 ou sel pharmaceutiquement acceptable de celui-ci, utilisé dans un procédé de traitement ou de prévention chez un sujet d'une maladie ou d'un état provoqué par un virus appartenant à la famille des Poxviridae ou associé à un tel virus, dans lequel R est choisi parmi soit des groupes oxa-alkyle substitués ou non substitués, soit des groupes alkyle substitués ou non substitués, soit des groupes cycloalkyle substitués ou non substitués, soit des groupes aryle substitués ou non substitués ; où dans lequel R représente 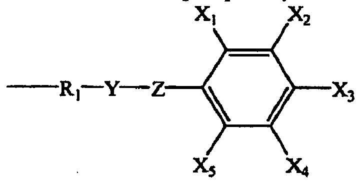
R₁ représente un groupe alkyle substitué ou non substitué ;
X₁₋₅ sont indépendamment choisis parmi H, NO₂, N₃ ou NH₂ ;
Y est absent ou représente un groupe alkyle en C₁ substitué ou non substitué, autre que carbonyle ; et
Z est choisi parmi une liaison ou NH ; sous réserve que quand Z représente une liaison, Y est absent, etsous réserve que quand Z représente NH, Y représente un groupe alkyle en C₁ substitué ou non substitué, autre que carbonyle ; et
dans lequel W₁₋₄ sont indépendamment choisis parmi l'hydrogène, des groupes alkyle substitués ou non substitués, des groupes halogénoalkyle substitués ou non substitués, des groupes alcanoyle substitués ou non substitués, des groupes aroyle substitués ou non substitués ou des groupes halogénoalcanoyle substitués ou non substitués.

2. Composé à utiliser de la revendication 1, dans lequel chacun parmi W₁, W₂, W₃ et W₄ représente l'hydrogène.

3. Composé à utiliser de la revendication 1 ou la revendication 2, dans lequel R est choisi parmi des groupes oxa-alkyle substitués ou non substitués, des groupes alkyle substitués ou non substitués, des groupes cycloalkyle substitués ou non substitués et des groupes aryle substitués ou non substitués.

4. Composé à utiliser de l'une quelconque des revendications 1 à 3, dans lequel R représente un groupe oxalkyle ou alkyle en C₆₋₁₂.

5. Composé à utiliser de la revendication 4, dans lequel R représente un groupe oxa-alkyle ou alkyle en C8-10.

6. Composé à utiliser de la revendication 1, dans lequel ledit composé est (i) la N-(9)-méthoxynonyl)désoxynojirimycine ou un sel pharmaceutiquement acceptable de celle-ci ; (ii) la N-(N-{4'-azido-2'-nitrophényl}-6-aminohexyl)désoxynojirimycine ou un sel pharmaceutiquement acceptable de celle-ci ; (iii) la N-(7-oxadécyl)désoxynojirimycine ou un sel pharmaceutiquement acceptable de celle-ci, ou (iv) la N-nonyl désoxynojirimycine ou un sel pharmaceutiquement acceptable de celle-ci.

7. Composé à utiliser de la revendication 1 ou la revendication 2, dans lequel R représente

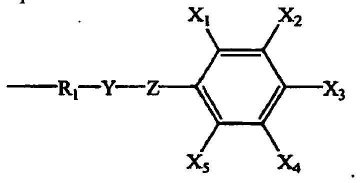

8. Composé à utiliser de la revendication 7, dans lequel X₁ représente NO₂ et où X₃ représente N₃.

9. Composé à utiliser de la revendication 7, dans lequel chacun de X₂, X₄ et X₅ représente l'hydrogène.

10. Composé à utiliser de l'une quelconque des revendications 1 à 9, dans lequel le sujet est un mammifère.

11. Composé à utiliser de la revendication 10, dans lequel le sujet est un être humain.

12. Composé à utiliser de l'une quelconque des revendications 1 à 9, la maladie ou l'état étant une variole.

13. Composé à utiliser de l'une quelconque des revendications 1 à 12, le virus appartenant à la famille des orthopoxvirus.

14. Composé à utiliser de l'une quelconque des revendications 1 à 13, le virus étant le virus de la variole.

15. Composé à utiliser de l'une quelconque des revendications 1 à 11 et 13, le virus étant le virus de la vaccine ou un virus de la cowpox.
